# EUROPEAN PATENT SPECIFICATION

(11) **EP 3 135 235 B1**
(45) Date of publication and mention of the grant of the patent: **19.02.2020**
(21) Application number: 16186378.2
(22) Date of filing: 30.08.2016
(51) Int. Cl.: A61B 17/29, A61B 18/14, A61B 17/00, A61B 18/00

(54) **LAPAROSCOPIC SURGICAL INSTRUMENT**
LAPAROSKOPISCHES CHIRURGISCHES INSTRUMENT
INSTRUMENT CHIRURGICAL LAPAROSCOPIQUE

(30) Priority: 31.08.2015 US 201562212169 P
(43) Date of publication of application: 01.03.2017
(73) Proprietor: Eubanks, Steve, Winter Park, FL 32789 (US)
(72) Inventor: Eubanks, Steve, Winter Park, FL 32789 (US)
(74) Representative: Ashton, Timothy

(56) References cited:
- EP-A1- 0 813 843
- EP-A2- 2 675 371
- US-A1- 2001 025 177
- M.A. VAN VEELEN ET AL: "New Ergonomic Design Criteria for Handles of Laparoscopic Dissection Forceps", JOURNAL OF LAPAROENDOSCOPIC & ADVANCED SURGICAL TECHNIQUES, vol. 11, no. 1, 1 February 2001 (2001-02-01), pages 17-26, XP055430815, US ISSN: 1092-6429, DOI: 10.1089/10926420150502896

## Description

### Related Applications

This application claims priority to United States Provisional Patent Application No. 62/212,169, filed August 31, 2015.

### Background

The present disclosure relates to surgical devices including improved minimally invasive or laparoscopic instruments. Although many laparoscopic instruments have been developed, certain features of such devices can be improved to make the device more ergonomic and easier to operate by permitting better hand positioning and gripping, better control of device components, and improved aesthetics. M.A. van Veelen et al. in "New Ergonomic Design Criteria for Handles of Laparoscopic Dissection Forceps" published in the journal of laparoendoscopic & advanced surgical techniques (vol. 11, no. 1, 1 February 2001 (2001-02-01), pages 17-26) have provided a comparison of ergonomic criteria of different types of handle designs.

### Summary

The minimally invasive or laparoscopic instruments described provide improvements in a variety of ways. The instruments of the present disclosure have larger openings in the grasping handles to allow multiple digits of a user to grasp the instrument. In addition, proximally directed extensions of a rotatable control allow a user with smaller hands to reach the control using the same hand that grasps the handle. Furthermore, a specially designed connection for cautery or other instruments is provided that can allow more flexibility in controlling the position of connecting wires, thereby facilitating operation. Additional stylistic and ergonomic features produce improved visual appeal.

In one embodiment, a surgical instrument is provided. The instrument comprising a handle, including an upper grasping section and a lower grasping section. The upper and lower grasping sections are joined at a pivot joint connected to a bearing that permits the upper grasping section to pivot towards and away from the lower grasping section, wherein the upper grasping section and the lower grasping section each include an opening shaped and sized to receive at least one digit of a user, the lower grasping section being sized to receive two or more digits of a user. A rotatable control (50) is positioned proximate the handle engages with the bearing and has a shaft attachment configured to attach to an elongated shaft, the bearing and the shaft attachment defining an axis therethrough. The bearing and the lower grasping section are joined at a fixed oblique angle of between about 110° and 145° as measured between the axis through the bearing and the shaft attachment and the lower grasping section.

Also provided is an electrocautery connection for a laparoscopic instrument. The electrocautery connection can comprise a solid ring portion for rotatably engaging a laparoscopic instrument and a conductive connection extending from the ring portion.

### Brief Description of the Drawings

Fig. 1 illustrates a perspective view of a laparoscopic instrument, according to certain embodiments.
Fig. 2 is an exploded view of a handle region of a laparoscopic instrument, according to certain embodiments.
Fig. 3 is a top view of a handle region of a laparoscopic instrument, according to certain embodiments.
Fig. 4 is a side view of a handle region of a laparoscopic instrument, according to certain embodiments.
Fig. 5 is a front-end view of a handle region of a laparoscopic instrument, according to certain embodiments.
Fig. 6 is a partial perspective view of a laparoscopic instrument that includes an improved instrument (electrocautery) connection.
Fig. 7 is a perspective view of the instrument connection separated from the laparoscopic instrument.
Fig. 8A is a perspective view of the instrument of Fig. 6 demonstrating the rotatable movement of the instrument (electrocautery) connection.
Fig. 8B is another perspective view of the instrument of Fig. 6 demonstrating the rotatable movement of the instrument (electrocautery) connection.

### Description of Certain Exemplary Embodiments

Reference will now be made in detail to certain exemplary embodiments according to the present disclosure, certain examples of which are illustrated in the accompanying drawings. Wherever possible, the same reference numbers will be used throughout the drawings to refer to the same or like parts.

In this application, the use of the singular includes the plural unless specifically stated otherwise. In this application, the use of "or" means "and/or" unless stated otherwise. Furthermore, the use of the term "including", as well as other forms such as "included" and "includes", is not limiting.

Use of the word "distal" is intended to indicate portions of an object nearest the patient while the word "proximal" is intended to indicate portions of an object furthest from the patient or closest to an operator's grasping hand. Any range described herein will be understood to include the endpoints and all values between the endpoints.

The section headings used herein are for organizational purposes only and are not to be construed as limiting the subject matter described.

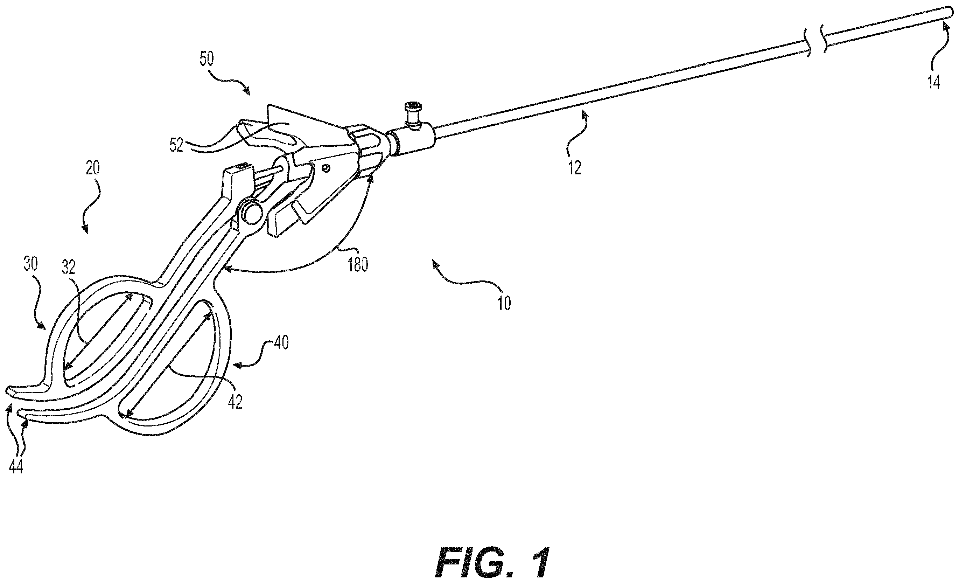

With reference to Fig. 1, a laparoscopic instrument 10 is shown according to various embodiments of the present invention. The laparoscopic instrument 10 can include a handle 20 attached to an elongated shaft 12. The handle 20 includes an upper grasping section 30 and a lower grasping section 40 that are ergonomically shaped to improve the user experience and ease of use of the instrument 10. A rotatable control 50 may be used to rotate the elongated shaft 12 relative to the handle 20.

The handle 20 includes an upper grasping section 30 and a lower grasping section 40. In accordance with various embodiments, the upper grasping section 30 can have an opening to accommodate a user's fingers and/or thumb. The opening can have an inner length 32 that is large enough to accommodate at least one and preferably more than one digit of a user simultaneously to improve the ability of the user to manipulate the tool and to improve user comfort. In accordance with various embodiments, the lower grasping section 40 can have an opening to accommodate a user's fingers. The opening can have an inner length 42 that is large enough to accommodate two or more digits of a user simultaneously to improve the ability of the user to manipulate the tool and to improve user comfort. The ends of the upper grasping section 30 and the lower grasping section 40 may include curved proximal extensions 44 to add to the visual appeal of the laparoscopic instrument 10. Similarly, the upper grasping section 30 and the lower grasping section 40 may be curved in a manner that is visually appealing. When a user grips the upper 30 and lower grasping sections 40, a linkage through the elongated shaft 12 can cause actuation of a tool located at the distal end 14 of the elongated shaft 12. The handle 20 may be attached to laparoscopic instruments including, but not limited to, scissors, graspers, electrocautery instruments, and retraction devices.

During an operation, the surgeon may be required to assume a less than optimal hand position to manipulate, grasp, or act upon the surgical site or control the device 10. To address this issue, a rotatable control 50 may be used to rotate the elongated shaft 12 relative to the handle 20. In embodiments of the present invention, the rotatable control 50 can have proximally directed extensions 52 placed around the knob to allow a user to manipulate the knob using a hand that is also gripping the handle 20. The use of the instrument 10 with a single hand enables the user to manipulate other objects with the free hand and thus improves efficiency during a surgical procedure. The proximally directed extensions 52 make it easier for users with smaller hands to reach and manipulate the rotatable control 50. In some embodiments, the rotatable control 50 can comprise one to six proximally directed extensions 52. The proximally directed extensions 52 can also be provided with a non-slip material along a portion or all of the surface of the extensions to improve manipulation by a user.

In some embodiments, the elongated shaft 12 and the handle 20 are joined at an oblique angle 180. The use of an oblique angle 180 improves the ergonomics of the instrument 10 and reduces strain on a user's hand. In some embodiments, the oblique angle 180 may be between about 110° and 145°. In a preferred embodiment, the oblique angle 180 is 136°. The combination of the oblique angle 180 and the rotatable control 50 may be designed to allow a user to obtain and maintain optimal ergonomics during use.

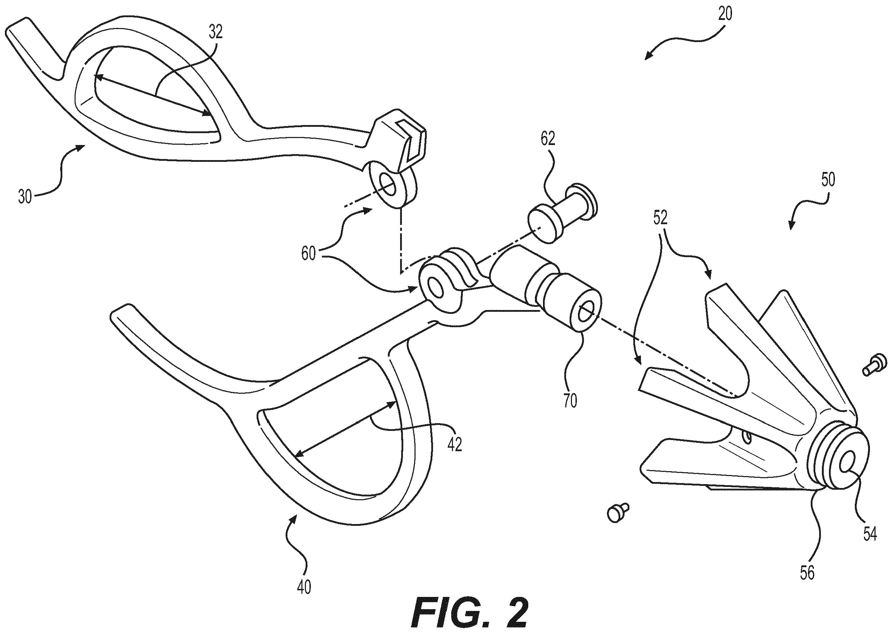

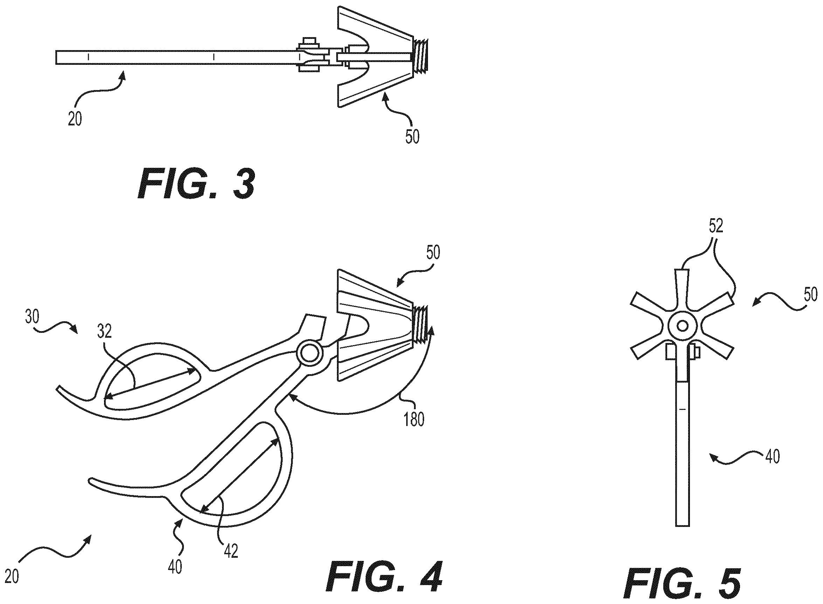

Turning now to Figs. 2-5, an exploded view and top, side, and front views are shown of a handle region of a laparoscopic instrument according to certain embodiments. The upper grasping region 30 and the lower grasping region 40 may be joined together at a joint 60. In some embodiments, the members of the joint 60 can be a clevis and prong, and the joint 60 may be affixed using a clevis pin 62. A rotatable control 50 can be positioned proximate the handle using set screws that connect to a bearing 70. A shaft attachment 54 can include threads 56 and may be located distally on the rotatable portion 50. The shaft attachment 54 allows the handle 20 to attach to an elongated shaft 12. In accordance with various embodiments, the bearing 70 and shaft attachment 54 can have holes to allow the passage of the shaft assembly.

The handle 20 or rotatable portion 50 can include a unique finish to provide a smooth, durable, and visually appealing instrument 10. The desired finish may be chosen depending on the material needed or the characteristics desired. In accordance with various embodiments, the materials and finish on the handle 20 or rotatable portion 50 can include, but are not limited to, oxide coating over steel, powder coat over steel, anodized titanium, injection-molded plastic, or any other material or finish required based upon application-specific considerations.

In some embodiments, the instruments described herein can further include one or more specially designed connectors. For example, laparoscopic instruments can include connections for cautery, including mono-polar electrocautery. The connections, which are necessarily attached to a wire or conductive power supply, however, can interfere with device movement, manipulation, or visualization. Accordingly, the present application also provides for connectors that allow better manipulation and flexibility of movement of connections such as mono-polar electrocautery connectors.

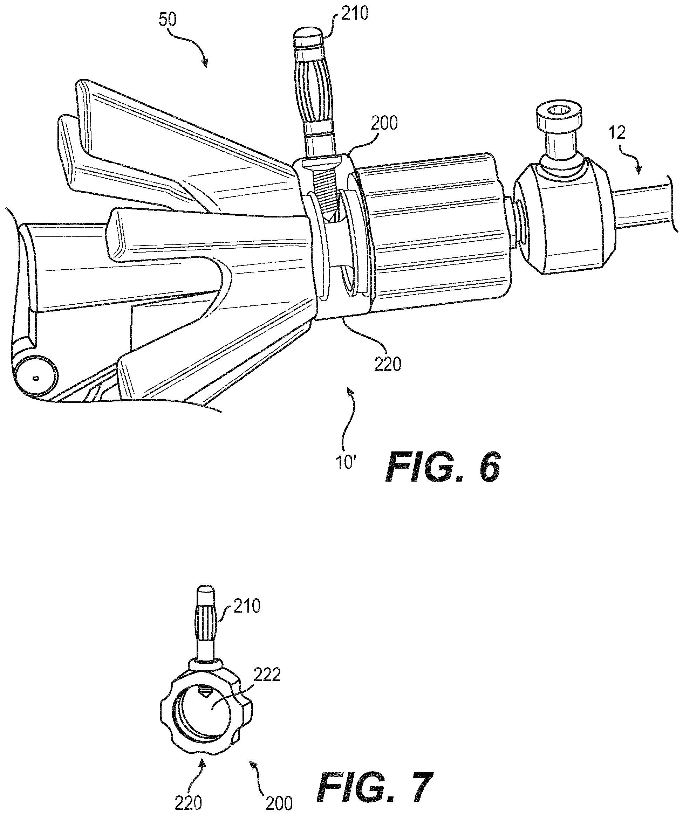

Fig. 6 is a partial perspective view of a laparoscopic instrument 10' that includes an improved instrument (electrocautery) connection 200, and Fig. 7 is a perspective view of the instrument connection 200 separated from the laparoscopic instrument. A portion of the connection 200 in Fig. 6 is partially transparent to show structures. As shown, the instrument 10' can include a handle and laparoscopic instruments as discussed above, but the connection 200 may alternatively be incorporated into other laparoscopic instruments or handles.

As shown, the connection 200 comprises a solid ring portion 220 with an opening 222 for rotatably engaging a laparoscopic instrument and a conductive connection 210 extending from the ring portion. The conductive connection 210 can include a connector for a monopolar electrocautery system, but could include a connection for other types of laparoscopic instruments or controls.

As shown, the ring portion 220 is positioned adjacent the rotatable control 50 or between the control 50 and shaft 12. Furthermore, the ring portion 220 surrounds a distal section of the handle 10' or shaft attachment 54 to provide a rotatable connection. It will be appreciated that the shaft attachment 54 may be modified to accommodate the connection 200 while also allowing attachment of a shaft portion 12, e.g., by extending the attachment and including a smooth or otherwise modified surface for rotation of the connection 200.

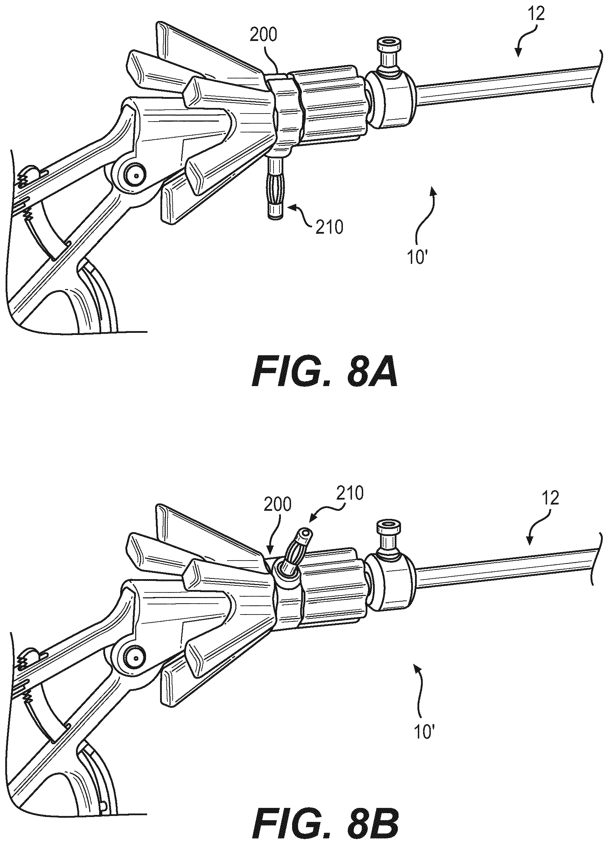

As noted, the connection 200 can be mobile or rotatable, thereby facilitating movement of connected wires or other devices. For example, Figs. 8A-8B are perspective views of the instrument 10' including a connection 200 positioned at different angles with respect to the handle 20 or shaft 12.

Other embodiments will be apparent to those skilled in the art from consideration of the specification and practice of the devices disclosed herein. The invention being defined by the appended claims.

## Claims

1. A surgical instrument, comprising:
a handle (20), including an upper grasping section (30) and a lower grasping section (40), the upper and lower grasping sections joined at a pivot joint (60) connected to a bearing (70) that permits the upper grasping section (30) to pivot towards and away from the lower grasping section (40), wherein the upper grasping section and the lower grasping section each include an opening shaped and sized to receive at least one digit of a user, wherein a rotatable control (50) positioned proximate the handle engages with the bearing and has a shaft attachment (54) configured to attach to an elongated shaft (12), the bearing and the shaft attachment defining an axis therethrough, and wherein the lower grasping section is sized to receive two or more digits of a user,
**characterized in that** the bearing and the lower grasping section are joined at a fixed oblique angle (180) of between about 110° and 145° as measured between the axis through the bearing and the shaft attachment and the lower grasping section (40).

2. The instrument of claim 1, further comprising the elongated shaft extending from the handle.

3. The instrument of any one of the preceding claims, wherein the rotatable control includes one or more elongated proximally directed extensions (52).

4. The instrument of claim 3, comprising six proximally directed extensions (52).

5. The instrument of any one of the preceding claims, wherein the grasping sections have a curved shape.

6. The instrument of any one of the preceding claims, further comprising a curved proximal extension (44) on the end of each grasping section.

7. The instrument of claim 3 or claim 4, further comprising an electrocautery connection (200) positioned adjacent to the rotatable control (50).

8. The instrument of claim 7, wherein the electrocautery connection is rotatable.

9. The instrument of claim 8, wherein the electrocautery connection includes a ring portion (220) and a conductive connection (210) extending from the ring portion.

## Patentansprüche

1. Chirurgisches Instrument, umfassend:
einen Griff (20), der einen oberen Greifabschnitt (30) und einen unteren Greifabschnitt (40) beinhaltet, wobei der obere und der untere Greifabschnitt an einem Drehgelenk (60) verbunden sind, das mit einem Lager (70) verbunden ist, das gestattet, dass der obere Greifabschnitt (30) hin zu und weg von dem unteren Greifabschnitt (40) schwenkt, wobei der obere Greifabschnitt und der untere Greifabschnitt jeweils eine Öffnung beinhalten, die geformt und bemaßt ist, um mindestens einen Finger eines Benutzers aufzunehmen, wobei ein nahe dem Griff positionierter Drehregler (50) das Lager in Eingriff nimmt und einen Wellenanschlussteil (54) aufweist, der für die Anbringung an einer länglichen Welle (12) konfiguriert ist, wobei das Lager und der Wellenanschlussteil eine hindurchgehende Achse definieren, und wobei der untere Greifabschnitt bemaßt ist, um zwei oder mehr Finger eines Benutzers aufzunehmen,
**dadurch gekennzeichnet, dass** das Lager und der untere Greifabschnitt unter einem festen schrägen Winkel (180) zwischen etwa 110 ° und 145 °, gemessen zwischen der Achse durch das Lager und dem Wellenanschlussteil und dem unteren Greifabschnitt (40), verbunden sind.

2. Instrument nach Anspruch 1, ferner umfassend die sich von dem Griff erstreckende längliche Welle.

3. Instrument nach einem der vorhergehenden Ansprüche, wobei der Drehregler eine oder mehrere längliche proximal gerichtete Verlängerungen (52) beinhaltet.

4. Instrument nach Anspruch 3, umfassend sechs proximal gerichtete Verlängerungen (52).

5. Instrument nach einem der vorhergehenden Ansprüche, wobei die Greifabschnitte eine gekrümmte Form aufweisen.

6. Instrument nach einem der vorhergehenden Ansprüche, ferner umfassend eine gekrümmte proximale Verlängerung (44) an dem Ende jedes Greifabschnitts.

7. Instrument nach Anspruch 3 oder Anspruch 4, ferner umfassend eine neben dem Drehregler (50) positionierte Elektrokauterisierungsverbindung (200).

8. Instrument nach Anspruch 7, wobei die Elektrokauterisierungsverbindung drehbar ist.

9. Instrument nach Anspruch 8, wobei die Elektrokauterisierungsverbindung einen Ringteil (220) und eine sich von dem Ringteil erstreckende leitende Verbindung (210) beinhaltet.

## Revendications

1. Instrument chirurgical, comprenant :
une poignée (20), incluant une section de préhension supérieure (30) et une section de préhension inférieure (40), les sections de préhension supérieure et inférieure étant jointes à une articulation à pivot (60) raccordée à un palier (70) qui permet à la section de préhension supérieure (30) de pivoter en allant vers et en revenant de la section de préhension inférieure (40), dans lequel la section de préhension supérieure et la section de préhension inférieure incluent chacune une ouverture formée et dimensionnée pour recevoir au moins un doigt d'un utilisateur, dans lequel une commande rotative (50) positionnée à proximité de la poignée s'engage avec le palier et une fixation d'arbre (54) configurée pour se fixer à un arbre allongé (12), le palier et la fixation d'arbre définissant un axe les traversant, et dans lequel la section de préhension inférieure est dimensionnée pour recevoir deux ou plus de deux doigts d'un utilisateur,
**caractérisé en ce que** le palier et la section de préhension inférieure sont joints à un angle oblique fixe (180) compris entre 110° et 145°, mesuré entre l'axe à travers le palier et la fixation d'arbre et la section de préhension inférieure (40).

2. Instrument selon la revendication 1, comprenant en outre l'arbre allongé s'étendant de la poignée.

3. Instrument selon l'une quelconque des revendications précédentes, dans lequel la commande rotative inclut une ou plusieurs extensions allongées orientées dans le sens proximal (52).

4. Instrument selon la revendication 3, comprenant six extensions orientées dans le sens proximal (52).

5. Instrument selon l'une quelconque des revendications précédentes, dans lequel les sections de préhension ont une forme incurvée.

6. Instrument selon l'une quelconque des revendications précédentes, comprenant en outre une extension proximale incurvée (44) sur l'extrémité de chaque section de préhension.

7. Instrument selon la revendication 3 ou la revendication 4, comprenant en outre une connexion d'électrocautérisation (200) positionnée adjacente à la commande rotative (50).

8. Instrument selon la revendication 7, dans lequel la connexion d'électrocautérisation peut tourner.

9. Instrument selon la revendication 8, dans lequel la connexion d'électrocautérisation inclut une partie annulaire (220) et une connexion conductrice (210) s'étendant de la partie annulaire.
